(19) Europäisches Patentamt
European Patent Office
Office européen des brevets

(11) **EP 3 805 785 A1**

(12) **DEMANDE DE BREVET EUROPEEN**

(43) Date de publication:
**14.04.2021 Bulletin 2021/15**

(51) Int Cl.:
**G01S 7/41** (2006.01)   **A61B 5/00** (2006.01)
**G01S 13/88** (2006.01)

(21) Numéro de dépôt: **19306308.8**

(22) Date de dépôt: **07.10.2019**

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**
Etats d'extension désignés:
**BA ME**
Etats de validation désignés:
**KH MA MD TN**

(71) Demandeurs:
- **Centre National de la Recherche Scientifique
  75016 Paris (FR)**
- **ENSEA - Ecole Nationale Supérieure de
  L'Electronique et de ses Applications
  95800 Cergy (FR)**
- **Université Cergy-Pontoise
  95000 Cergy (FR)**

- **The University Court of the University
  of Glasgow
  Glasgow G12 8QQ (GB)**

(72) Inventeurs:
- **ROMAIN, Olivier
  91230 Montgeron (FR)**
- **LEKERNEC, Julien
  29300 Quimperle (FR)**
- **LORANDEL, Jordane
  78700 Conflans-sainte-Honorine (FR)**
- **FIORANELLI, Francesco
  Glasgow, G11 6ED (GB)**

(74) Mandataire: **A.P.I. Conseil
Immeuble Newton
4, rue Jules Ferry
64000 Pau (FR)**

(54) **DISPOSITIF DE CARACTÉRISATION DE L'ACTIMÉTRIE D'UN SUJET EN TEMPS RÉEL**

(57) L'invention présente un dispositif (1) pour caractériser en temps réel l'actimétrie d'un sujet, présentant : un radar (2) émettant et recevant des signaux radar et présentant une interface logicielle pour configurer la forme du signal émis ; des moyens de traitement et de calcul (3) couplés au radar (2), présentant un classifieur (3a) entrainé à l'aide d'une base de données, lesdits moyens de traitement et de calcul (3) étant configurés pour réaliser en temps réel: -une acquisition d'images micro-doppler (6) couleurs ayant plusieurs canaux de couleurs (R, V, B), présentant chacune des signatures micro-doppler (6a) avec des pixels couleurs dont la valeur est fonction d'une réflectivité et d'une vitesse du sujet; -un traitement des images micro-doppler (6) pour : calculer une image dite monochromatique ayant des pixels monochromatiques, ayant chacun une intensité monochromatique donnée, à partir des pixels couleurs de chaque image micro-doppler couleur ; transformation de l'image monochromatique en image binaire par segmentation, selon un seuil d'intensité, des pixels monochromatiques, réalisant des pixels binaires dont la valeur est dépendante de l'intensité chromatique du pixel monochromatique associé au pixel binaire, par rapport au seuil,

Figure 1

EP 3 805 785 A1

**Description**

**DOMAINE DE L'INVENTION**

La présente invention concerne un dispositif pour caractériser en temps réel l'actimétrie d'un sujet.

**ETAT DE LA TECHNIQUE**

La détection automatique de posture a donné lieu ces dernières années à une intense activité de recherche et des grandes retombées économique. Les capteurs 3D type en anglais « Kinect » en particulier ont permis de changer de paradigme dans le jeu en offrant la possibilité de prendre en compte l'information de profondeur (3D) et ainsi de discriminer de manière plus efficace les différents types de mouvements des sujets.

On retrouve aujourd'hui, la plupart de ces systèmes de mesure et d'analyse des mouvements tant dans le milieu de l'animation virtuelle (simulation de la marche) que dans le domaine biomécanique et médical.

Ces systèmes permettent d'étudier des problématiques de détection de pathologie du sujet, d'analyser le corps en activité, ou, de comprendre les mécanismes de la marche.

Les compétitions académiques encore récemment organisés sur cette thématique montrent néanmoins les limitations des capteurs actuels, notamment pour la reconnaissance de posture dans diverses situations et en particulier les chutes de sujets déficients.

Les systèmes actuels peuvent être regroupés en trois catégories ; les systèmes embarqués sur un sujet, les systèmes déportés et, les systèmes hybrides (mixte d'embarqués et de déportés).

De nombreuses technologies embarquées ont été proposées pour la surveillance des sujets et spécifiquement pour la détection des chutes [3], ces trente dernières années. Celles-ci incluent les dispositifs portables tels que les podomètres, locomètres, accéléromètres, les gyroscopes et les boutons-poussoirs de panique, les capteurs inertiels tels que les smartphones, les capteurs infrarouges, vibratoires, acoustiques, magnétiques, ...

Bien que ces dispositifs donnent de bons résultats [4] sur l'identification de chutes (98%), la plupart de ces solutions embarquées sur le sujet souffrent de plusieurs problèmes majeurs [2] qui limitent leurs usages :

- Ils doivent être portés, ce qui dépend de la conformité de l'utilisateur ou d'y penser si vous vous réveillez la nuit pour aller aux toilettes.
- Ils sont facilement cassables en cas de chute, de choc ou si on s'assoie dessus.
- Ils doivent être rechargés, ce qui peut être difficile pour les sujets ayant une déficience cognitive.
- Le taux de fausses alarmes
- Ils sont stigmatisant pour les sujets
- Ils ne respectent pas (pour certains) la vie privée.

Les dispositifs déportés sont généralement basés sur l'utilisation de systèmes de mesure intégrés dans le lieu de vie de l'usager. On retrouve l'emploi de caméra(s) vidéo [5], les tapis roulants de marche, les capteurs photographiques RGB-D pour « Red, Green, Blue detector » selon une terminologie anglo-saxonne et les radars ou une combinaison de ces systèmes. Des appartements complets peuvent être équipés de capteurs de mouvement PIR pour « passive infrared sensor » selon une terminologie anglo-saxonne, de capteurs de gazinière, des capteurs dans le lit, des capteurs au sol ..., ce qui permet de donner des schémas d'activités de la vie quotidienne. Cependant, ils ne sont pas capables de donner des informations plus fines sur l'analyse de la marche pour la détection des changements.

Pour les systèmes radar et RGBI-D, des défis ouverts restent à relever afin de déployer et d'utiliser ces systèmes dans des scénarios pratiques à domicile ou dans des établissements spécialisés :

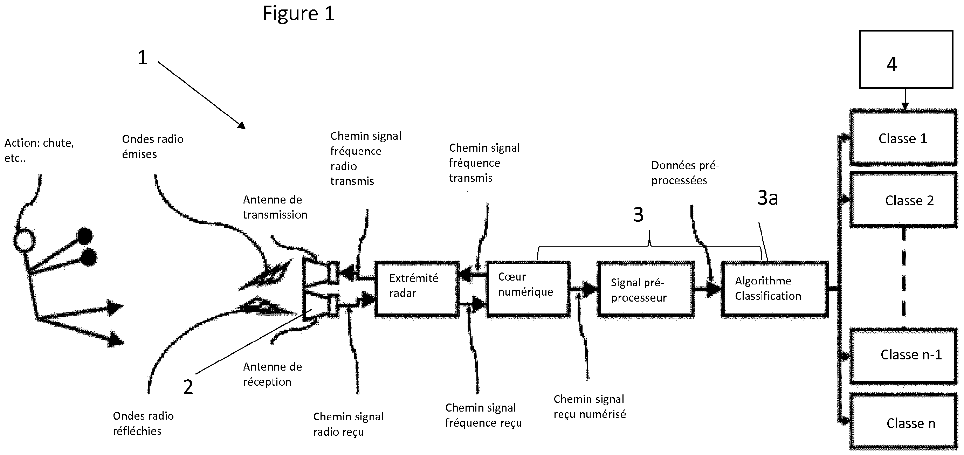

- Concernant les caméras [6], les principaux défis à relever restent les occlusions (zones de pixels mortes), le fonctionnement de nuit, les zones de pixels mortes en 3D, la précision, la résolution des caméras, et le respect de la vie privée. La surveillance des sujets dans leur vie quotidienne pose un réel problème de confidentialité (figure 1). En effet, en fonction du capteur, la perception de l'intrusion et du respect de la vie privée sont différents. Cette perception se traduit par une modification des comportements et l'ajout de
- Concernant les systèmes radar [7], l'environnement intérieur susceptibles de générer des cibles multi-trajet et la réglementation des émissions.

Bien qu'il y ait plus de défis technologiques avec le radar, le fait qu'il n'y ait pas de problème juridique concernant les droits d'image et qu'aucune image de sujet ne soit prise, respectant ainsi la vie privée, facilite l'acceptation des utilisateurs finaux et des investisseurs. Pour les raisons susmentionnées, la modalité radar est une piste de recherche intéressante encore inexploitée en milieu spécialisé (EHPAD), en milieu carcéral ou dans les smart homes.

Le radar est considéré comme une technologie émergente pour la surveillance de la santé et la détection des chutes dans la vie assistée en raison d'un certain nombre d'attributs non partagés par d'autres modalités de détection. La méthode la plus courante pour classifier les activités est basée sur l'extraction de caractéristiques issues des signatures micro-Doppler (spectrogramme). Le mouvement relatif des composantes structurelles d'un objet / corps génère des zones de pixels uniques dans le domaine temps-fréquence des retours radar. Par conséquent, différentes activités génèrent des caractéristiques distinctives uniques dans les signatures micro-Doppler qui peuvent être utilisées pour la classification. En général, les individus mesurent et extraient différentes caractéristiques des spectrogrammes (temps lent, Doppler), suivies de différentes classifications automatiques. Les techniques pour la classification automatique comprennent l'analyse discriminante de Fisher (FDA), les voisins K-plus proches (KNN), la classification Naïve Bayésienne (NB), et les machines à vecteurs de support (SVM).

Récemment, avec l'augmentation de la puissance de calcul, il est devenu possible d'utiliser des méthodes de « deep learning » selon une terminologie anglo-saxonne. Le « deep learning », ou apprentissage profond, regroupe l'ensemble des méthodes d'apprentissage automatique, supervisées ou non-supervisées, et est capable de déterminer automatiquement les caractéristiques les plus pertinentes à des fins de classification.

Par exemple, pour reconnaître un visage en vision par ordinateur avec les réseaux convolutionnels de neurones (CNN), la première couche peut reconnaître les bords à différents angles, puis dans la seconde couche différentes parties du visage (yeux, bouche du nez), puis superposer des faces entières et essayer ensuite de classifieur ce qu'il voit.

Une autre classe d'architectures d'apprentissage en profondeur utilisées pour le traitement de la parole naturelle sont les réseaux neuronaux récurrents (RNN) avec des unités récurrentes bloquées et la mémoire à long terme (LST).

La plupart de ces approches permettent de démontrer que le traitement des signaux issus des spectrogrammes d'un radar rend possible la détection des activités humaines. Les résultats obtenus sont issus de traitement offline sur des bases de données.

Les dimensions embarquées et implémentation temps-réel des traitements ne sont pas abordées. Celles-ci nécessitent de concevoir les algorithmes de classification en prenant en compte dès la conception des contraintes de temps de calcul (débits IO), d'implémentation efficace des traitements en respectant des contraintes supplémentaires de consommation.

## EXPOSE DE L'INVENTION

Pour répondre à ces objectifs, un nouveau système basé sur le développement d'une architecture de radar logiciel émettant à la fréquence (bande 2-4GHz) a été développé (figure 1).

Le caractère logiciel du radar rend flexible la forme d'onde qui est émise ainsi que le traitement des signaux, au pied de l'antenne. Ainsi, le traitement des signatures radars de type micro-doppler par des solutions d'algorithmes de traitement d'images et de « machine learning » selon une terminologie anglo-saxonne ou d'apprentissage automatique, permet de caractériser l'actimétrie d'un sujet.

L'invention repose sur l'agrégation de plusieurs techniques en vue de répondre à une problématique. Le caractère inventif comprend deux parties :

- Une technique d'extraction de paramètres de forme issue de technique de vision par ordinateur, sur des images de type spectrogrammes micro-doppler à hautes résolutions représentant des cartes de vitesse / distance.
- Une technique de classification binaire de type SVM.

L'utilisation conjointe de ces deux techniques, permet d'obtenir des performances à l'état de l'art (approches « deep ») tout en garantissant une implémentation matérielle plus faible (compatible avec un objet connecté de faible puissance de calcul) et une consommation moindre.

## DESCRIPTION DES FIGURES

D'autres objectifs, caractéristiques et avantages sortiront de la description détaillée qui suit en référence aux dessins donnés à titre illustratif et non limitatif parmi lesquels :

- la figure 1 représente un schéma de principe de l'invention ;
- la figure 2 représente des signatures micro-doppler qui vont être traitées par le dispositif et procédé selon l'invention ; les signatures micro-dopplers sont les suivantes : (a) Assis sur une chaise ; (b) Levé d'une chaise ; (c) Flexion et récupération d'un stylo à terre ; (d) Flexion et lace ses lacets ; (e) Tomber en avant ; (f) Accroupissement pour regarder au-dessus et en dessous d'un meuble

- la figure 3 représente une chaîne algorithmique de traitement des signatures micro-doppler et qui fait l'objet de la présente invention ;

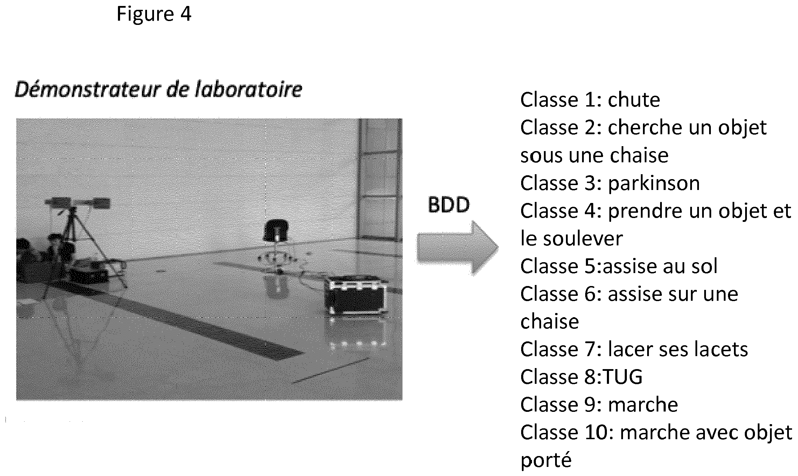

- la figure 4 représente une preuve de concept de laboratoire et Classes considérées;

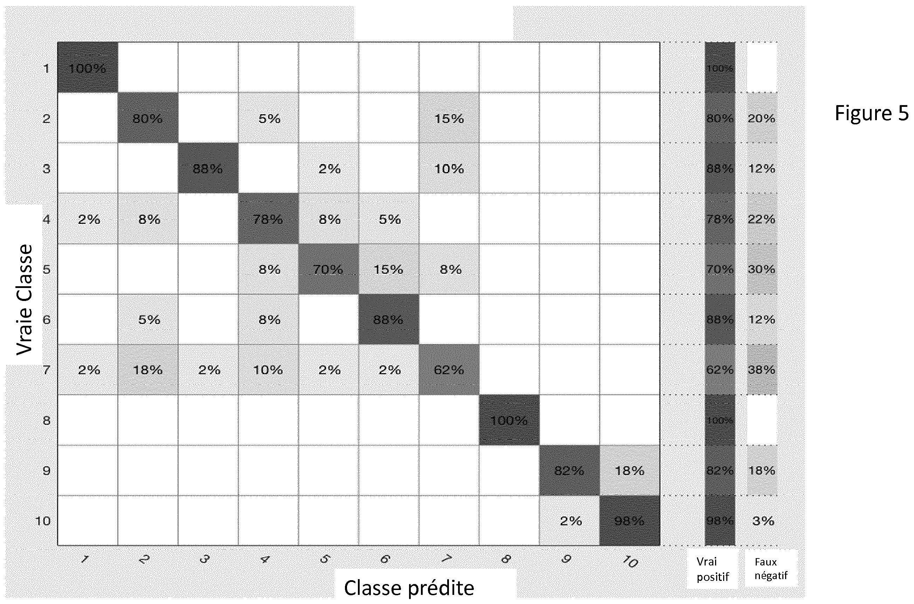

- la figure 5 représente une matrice de confusion.

## DESCRIPTION GENERALE DE L'INVENTION

La présente invention présente un dispositif 1 pour caractériser en temps réel l'actimétrie d'un sujet, présentant :

- un radar 2 émettant et recevant des signaux radar 2, et présentant une interface logicielle pour configurer la forme du signal émis ;

- des moyens de traitement et de calcul 3 couplés au radar 2, présentant un classifieur 3a entrainé à l'aide d'une base de données.

Lesdits moyens de traitement et de calcul 3 sont configurés pour réaliser en temps réel:

- une acquisition d'images micro-doppler 6 couleurs ayant plusieurs canaux de couleurs (R, V, B), présentant chacune des signatures micro-doppler 6a avec des pixels couleurs dont les valeurs d'intensités se répartissent sur une échelle graduelle;

- un traitement des images micro-doppler 6 pour :

    ✓ calculer une image dite monochromatique ayant des pixels monochromatiques, ayant chacun une intensité monochromatique donnée, à partir des pixels couleurs de chaque image micro-doppler couleur ;
    ✓ transformation de l'image monochromatique en image binaire par segmentation, selon un seuil d'intensité, des pixels monochromatiques,
    réalisant des pixels binaires, dont la valeur binaire pour chaque pixel binaire est fonction de la valeur de l'intensité chromatique du pixel monochromatique associé ou correspondant au pixel binaire (à la même position sur l'image binaire que la position du pixel monochromatique sur l'image chromatique), par rapport à la valeur du seuil,
    formant, sur la surface de l'image binaire, des zones segmentées 5a qui présentent des pixels binaires de même valeur binaire, et qui sont issues de la transformation de chaque signature micro-doppler 6a;

- un calcul de valeurs de paramètres sur chaque zone segmentée 5a, chaque paramètre étant uniquement une caractéristique de forme des zones segmentées 5a ;

- un classement de chaque image binaire 5 dans une classe se rapportant à l'actimétrie du sujet, en fonction des valeurs des paramètres calculés pour toutes les zones segmentées 5a de l'image binaire 5, à l'aide du classifieur 3a entraîné.

Les pixels couleurs des signatures micro-doppler ont une intensité dont la valeur est fonction d'une réflectivité et d'une vitesse du sujet, et se répartit sur une échelle graduelle (au sens de continu).

Ainsi, la conversion de l'image doppler couleur en l'image monochromatique peut consister à convertir, pour chaque pixel de la représentation doppler couleur, le triplet de valeurs représentant les niveaux des couleurs primaires de chaque pixel couleur en une valeur entière qui en la somme, et représentant une luminosité ou une intensité lumineuse ou luminance associée à un pixel de représentation ainsi produite. Chaque zone segmentée présente ainsi des premiers pixels binaires de même valeur et qui se distingue du fond de la surface de l'image binaire.

En effet le fond de la surface de l'image binaire présente lui aussi des seconds pixels binaires de même valeur mais d'une valeur différente de celle des premiers pixels binaires.

Ainsi les signatures micro-doppler se trouvent segmentées en plusieurs zones segmentées.

La présente invention vise à éviter de faire les calculs de l'état de l'art sur les valeurs représentées dans les signatures micro-doppler couteuses en temps, et ne se porte qu'uniquement sur les caractéristiques de forme des zones segmentées. La transformation par la binarisation des signatures micro-doppler passe d'une information 3D à une information 2D mais permet de faire des calculs rapides et est efficace pour classer les images.

Le présent dispositif 1 présente des moyens de stockage 4 couplés aux moyens de calcul 3 pour stocker le classifieur 3a, les images micro-doppler 6, les images monochromatiques, les images binaires 5 et le classement obtenu des images binaires 5.

Dans une réalisation, le calcul de l'image monochromatique est réalisé avec la couleur grise qui est fonction de la valeur des canaux de couleur (R, V, B) des pixels couleurs, par exemple en suivant la formule : Gris= 0.299*Rouge+0,587*Vert+0.144*Bleu, pour chaque pixel des images microdopplers couleurs qui a une valeur d'intensité rouge, une valeur d'intensité vert et une valeur d'intensité bleu.

Avantageusement, le dispositif 1 est configuré pour caractériser en continu l'actimétrie de la personne, la base de données étant alimentée et augmentée en continu avec le classement obtenu des images binaires 5 réalisé par le classifieur 3a.

Avantageusement, les moyens de traitement et de calcul 3 sont configurés pour filtrer les pixels de même valeur binaire que les pixels des zones segmentées, mais situés à distance des zones segmentées 5a.

Par exemple, il est utilisé un filtrage morphologique érosion et dilatation pour supprimer ces pixels isolés, qui sont situés à plusieurs pixels de distance de ces zones segmentées et qui visiblement ne font pas partie de ces dernières.

Les caractéristiques de forme de chaque zone segmentée 5a de pixels binaires de même valeur binaire, sont indépendantes des valeurs de fréquence, temps, vitesse, puissance, mesurées sur les signatures micro-doppler.

Avantageusement, le classifieur 3a des moyens de calcul 3, classe les images sans utiliser de réseaux de neurones, tels que des réseaux de neurones convolutionnels (CNN), ou des réseaux de neurones multicouches (DNN), trop couteux en temps.

Avantageusement, le dispositif 1 est embarqué, et le classifieur 3a est choisi notamment dans la liste suivante :

- un boosting algorithme ;
- une cascade adaboost ;
- apprentissage actif ("active learning" en terminologie anglo-saxonne) ;
- un classifieur 3a binaire (exemple SVM (en anglais « Support Vector Machine » à noyau cubique).

Le boosting regroupe un ensemble d'algorithmes tels que : Adaboost, LPBoost, TotalBoost, BrownBoost, xgboost, MadaBoost, LogitBoost. Le Boosting est une méthode séquentielle et chaque échantillon est tiré en fonction des performances de la règle de base sur l'échantillon précédent. Le Boosting et sa mise en œuvre sont décrits en détail dans Freund & Schapire 1999 (Machine Learning Large. Margin Classification Using the Perceptron Algorithm). Dans le cadre d'un apprentissage actif, un algorithme d'apprentissage est capable d'interroger de manière interactive l'utilisateur (ou une autre source d'informations) pour obtenir les sorties souhaitées à de nouveaux points de données.

Les caractéristiques de forme de chaque zone segmentée 5a de pixels binaires de même valeur sont choisies notamment parmi la liste suivante: surface, périmètre, centroïde de degré 1, de degré 2, orientation, etc.

De façon plus précise,

-

$$\text{Surface} = Surface = \sum_x \sum_y p_{x,y} \; avec \; p_{x,y} \; appartient \; \{forme \; i\}.$$

$P_{x,y}$ représente la valeur du pixel aux coordonnées x,y dans l'image. La surface comptabilise le nombre de pixel ayant la même valeur, '1', dans l'image. Avantageusement, la détection de contour est obtenue par un filtrage convolutionel par le noyau de
Sobel

- Périmètre = Somme des $p_{x,y}$ appartenant au pourtour de la surface. Le périmètre est obtenu en sommant tous les pixels de la même valeur sur le contour de la forme. Un opérateur de Sobel est utilisé pour définir le contour de la surface. Ainsi, l'équation suivante peut être utilisée :

$$Perimetre = \sum_x \sum_y \quad p_x \cdot p_y \; avec \; x,y \in \{bordure \; de \; la \; forme \; i\},$$

- Moment d'ordre 0 :

$$Mo = \sum_x \sum_y p_x^i \cdot p_y^j \; avec \; x,y \in \{forme \; i\} \; et \; i \; et \; j = 0$$

- Centroïde de degré 1 (moment d'ordre 1), correspondant au centre de masse de la forme. Il est de coordonnées $g_x$ et $g_y$, et, calculé à partir des équations suivantes :

$$gx = \frac{1}{N}\Sigma_{i=0}^{N} p_{x_i} \text{ et } gy = \frac{1}{N}\Sigma_{i=0}^{N} p_{y_i}$$

avec N le nombre de pixel total dans l'image, $p_{xi}$ l'abscisse du pixel et $p_{yi}$ l'ordonnée du pixel.

Il peut aussi être obtenu selon l'équation suivante :

$$M1 = \sum_x \sum_y p_x^i \cdot p_y^j \; avec \; x, y \in \{forme \; i\} \; et \; i \; et \; j = 1,$$

- Centroïde de degré 2, il est calculé par exemple à partir de l'équation suivante :

$$M2 = \sum_x \sum_y p_x^i \cdot p_y^j \; avec \; x, y \in \{forme \; i\} \; et \; i \; et \; j = 2$$

- Orientation : obtenue par la détermination de la droite passant par le moment d'ordre 1 et qui minimise la distance des points de contours à la droite.

Exemples :

- pour la surface : comptage des pixels blancs, moment d'ordre 0 (moyennes selon l'axe x et y) ;
- pour le périmètre : détection de contour (algorithme de Sobel) + comptage des pixels blancs ;
- pour le calcul des moments d'ordre 1 et 2 :

$$Mm,n = \; somme(somme(x^m y^n f(x,y)dxdy))$$

- Carré englobant ;
- Ellipse englobante.

Par exemple des polynômes de Legendre peuvent être utilisés.

Dans une réalisation, la segmentation des pixels est réalisée à l'aide d'un seuil binaire fixe.

Dans une autre réalisation, la segmentation des pixels est réalisée à l'aide d'un seuil binaire adaptatif en fonction de la luminance (exemple : dans la publication Otsu [9]).

Les classes peuvent être choisies parmi la liste suivante :

- marche,
- course
- levée,
- assise.

Les classes peuvent être choisies parmi la liste suivante :

- marche avec un objet porté avec 2 mains,
- chute,
- assise sur une chaise,
- lacer ses lacets,
- marche Parkinsonienne,
- assise au sol,
- ramasser un objet,
- chercher un objet sous une chaise,
- lever d'une chaise et marche.

Avantageusement, les moyens de calcul 3 sont configurés pour stocker les séquences successives de classe obtenues, et déterminer une carte d'activité du sujet, notamment en utilisant des chaines de Markov, des arbres de décision binaire ou pas, etc..

Dans une réalisation, le radar 2 est mono-statique (un émetteur et un récepteur).

Dans une autre réalisation, le radar 2 est bi-statique (un émetteur et plusieurs récepteurs).

Le radar 2 émet les signaux radar selon une bande de fréquence comprise entre 6 MHz et 250 GHz, de préférence entre 2 et 4 GHz, de façon plus préférée entre 2,3 et 2,5 GHz

La présente invention concerne aussi un système de contrôle de l'actimétrie d'un sujet, ledit système comprenant :

- un bâtiment avec des murs ;
- un dispositif 1 selon l'invention, ledit dispositif étant intégré dans un mur dudit bâtiment.

Le dispositif 1 peut être couplé avec un dispositif d'alerte et de contrôle, apte à alerter d'une nécessité d'intervention sur le sujet dont l'actimétrie est suivie, si la succession de classes des images binaires 5, déterminée en temps réel, indique comme carte d'activité un comportement à risque.

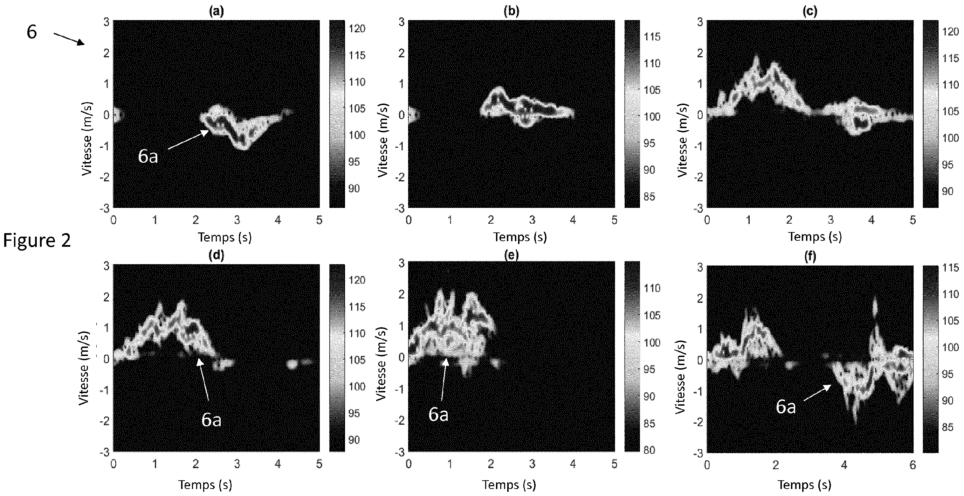

Des premiers traitements ont été développés pour la reconnaissance de l'actimétrie de sujet. Une base de données a été constituée à partir d'un prototype de radar 2 logiciel, composée de 10 classes (marche, marche avec un objet porté avec 2 mains, chute, assise sur une chaise, lace ses lacets, marche Parkinsonienne, assise au sol, ramasse un objet, cherche un objet sous une chaise, levée d'une chaise et marche), de 5 sujets. 10 caractéristiques sont extraites des images correspondant à des paramètres de formes (surface, périmètre, centroïde de degré 1, de degré 2, orientation, etc.). 70% des caractéristiques extraites ont été utilisées pour entrainer des modèles statistiques (SVM - Support Vector Machine à noyau cubique) (figure 2).

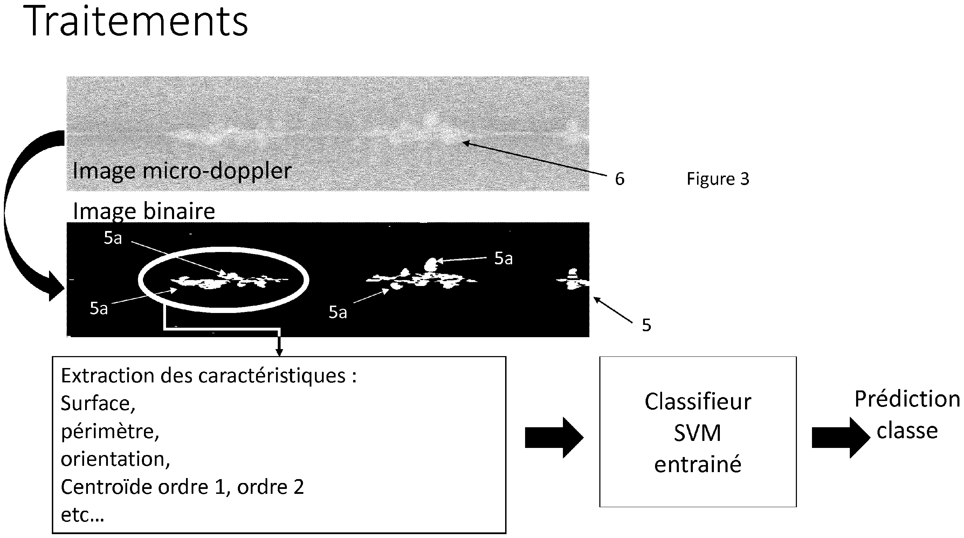

A partir de la base de données, la précision (en anglais « accuracy ») sur l'ensemble des 10 classes est de 84.5% (figure 3). Ces résultats sont comparables à ceux obtenus par des réseaux de neurones convolutionnels - CNN - mais avec une plus faible complexité algorithmique, les rendant plus optimisés pour une implémentation en temps réel.

## APPLICATIONS PRINCIPALES DE L'INVENTION

1. Détection des chutes à domicile, en EHPAD et en milieu hospitalier.

2. Aide à l'évaluation des déficiences de la marche pour les professionnels de la santé (Kinésithérapie par exemple).

La quantification de tests utilisés en routine clinique pour détecter d'éventuelle pathologie ou fragilité. Les trois principaux tests sont le TUG - en anglais « Time Up and Go », le test de l'appui unipodal et le test de Tinetti. Ces tests permettent de fournir une évaluation de la qualité de la marche et de l'équilibre du sujet pour ainsi évaluer son état. A partir des images des spectrogrammes, il devient possible d'extraire des métriques spécifiques et d'objectiver les mesures de TUG, notamment. Les premiers résultats permettent d'identifier un test de TUG avec 100% de précision. Pour cela, des métriques supplémentaires devront être définies pour extraire des qualités métrologiques du TUG.

L'observation répétée de l'activité d'un sujet au cours du temps permettra d'identifier les signes de dégradation de la marche. Après avoir identifié la classe de l'activité (marche, marche lente, ...) des métriques sur la marche peuvent être extraites des spectrogrammes pour caractériser son comportement biomécanique comme la longueur du pas, les vitesses de marche (min, max, moyenne), les asymétries spatio-temporelle, la cadence de marche et l'évolution du centre de gravité. Le suivi de ces paramètres permettra de détecter des variations de comportement et d'identifier des dégradations pouvant amener à des chutes.

3. Détection des suicides en milieu carcérale

Le suivi de l'activité en temps-réel du détenu à risques à partir de la solution ici présenté permettra d'anticiper les situations extrêmes de suicide (risque 7 fois plus élevé que dans le reste de la population) en identifiant des signatures caractéristiques de l'activité.

Publications citées :

[1] M. G. Amin, Y. D. Zhang, F. Ahmad, and K. C. D. Ho, "Radar 2 Signal Processing for Elderly Fall Détection: The future for in-home monitoring," IEEE Signal Processing Magazine, vol. 33, no. 2, pp. 71-80, 2016.
[2] (2012). Report to Congress: Aging services Technology Study.

[3] C. Debes, A. Merentitis, S. Sukhanov, M. Niessen, N. Frangiadakis, and A. Bauer, "Monitoring Activities of Daily Living in Smart Homes: Understanding human behavior," IEEE Signal Processing Magazine, vol. 33, no. 2, pp. 81-94, 2016.

[4] H. Li, A. Shrestha, F. Fioranelli, J. L. Kernec, and H. Heidari, "Multisensory Data Fusion for Human Activities Classification and Fall Détection," presented at the IEEE Sensors 2017, Glasgow, UK, 30 Oct - 1 Nov, 2017.

[5] E. Cippitelli, F. Fioranelli, E. Gambi, and S. Spinsante, "Radar 2 and RGB-Depth Sensors for Fall Détection: A Review," IEEE Sensors Journal, vol. 17, no. 12, pp. 3585-3604, 2017.

[6] E. Cippitelli, F. Fioranelli, E. Gambi, and S. Spinsante, "Radar 2 and RGB-Depth Sensors for Fall Détection: A Review," IEEE Sensors Journal, vol. 17, no. 12, pp. 3585-3604, 2017.

[7] M. G. Amin, Y. D. Zhang, F. Ahmad, and K. C. D. Ho, "Radar 2 Signal Processing for Elderly Fall Détection: The future for in-home monitoring," IEEE Signal Processing Magazine, vol. 33, no. 2, pp. 71-80, 2016.

[8] B. Jokanovic and M. Amin, "Fall Détection Using Deep Learning in Range-Doppler Radar 2s," IEEE Transactions on Aerospace and Electronic Systems, vol. PP, no. 99, pp. 1-1, 2017.

[9] Nobuyuki Otsu, « A threshold selection method from gray-level histograms », IEEE Trans. Sys., Man., Cyber., vol. 9, 1979, p. 62-66

## Revendications

**1.** Dispositif (1) pour caractériser en temps réel l'actimétrie d'un sujet, présentant :

• un radar (2) émettant et recevant des signaux radar et présentant une interface logicielle pour configurer la forme du signal émis ;
• des moyens de traitement et de calcul (3) couplés au radar (2), présentant un classifieur (3a) entrainé à l'aide d'une base de données,
lesdits moyens de traitement et de calcul (3) étant configurés pour réaliser en temps réel :

- une acquisition d'images micro-doppler (6) couleurs ayant plusieurs canaux de couleurs (R, V, B), présentant chacune des signatures micro-doppler (6a) avec des pixels couleurs;
- un traitement des images micro-doppler (6) pour :

✓ calculer une image dite monochromatique ayant des pixels monochromatiques, ayant chacun une intensité monochromatique donnée, à partir des pixels couleurs de chaque image micro-doppler couleur ;
✓ transformation de l'image monochromatique en image binaire par segmentation, selon un seuil d'intensité, des pixels monochromatiques,
réalisant des pixels binaires, dont la valeur binaire pour chaque pixel binaire est fonction de la valeur de l'intensité chromatique du pixel monochromatique associé au pixel binaire, par rapport à la valeur du seuil d'intensité,
formant, sur la surface de l'image binaire, des zones segmentées (5a) qui présentent des pixels binaires de même valeur binaire, et qui sont issues de la transformation de chaque signature micro-doppler (6a);

- un calcul de valeurs de paramètres sur chaque zone segmentée (5a), chaque paramètre étant uniquement une caractéristique de forme des zones segmentées (5a) ;
- un classement de chaque image binaire (5) dans une classe se rapportant à l'actimétrie du sujet, en fonction des valeurs des paramètres calculés pour toutes les zones segmentées (5a) de l'image binaire (5), à l'aide du classifieur (3a) entraîné,

• des moyens de stockage (4) couplés aux moyens de traitement et de calcul (3) pour stocker le classifieur (3a), les images micro-doppler (6), les images monochromatiques, les images binaires (5) et le classement obtenu des images binaires (5).

**2.** Dispositif (1) selon la revendication précédente, dans lequel le dispositif (1) est configuré pour caractériser en continu l'actimétrie du sujet, la base de données étant alimentée en continu avec le classement obtenu des images binaires (5) avec les paramètres de forme appliqués à chaque zone segmentée, et réalisé par le classifieur (3a), des images micro-doppler (6).

**3.** Dispositif (1) selon la revendication précédente, dans lequel les moyens de traitement et de calcul (3) sont configurés pour filtrer les pixels de même valeur binaire que les pixels des zones segmentées, mais situés à distance des zones segmentées (5a).

**4.** Dispositif (1) selon l'une quelconque des revendications précédentes, dans lequel les caractéristiques de forme de chaque zone segmentée (5a) de pixels binaires sont indépendantes des valeurs de fréquence, temps, vitesse, puissance, mesurées sur les signatures micro-doppler.

**5.** Dispositif (1) selon l'une des revendications précédentes, dans lequel le classifieur (3a) des moyens de calcul (3), classe les images binaires (5) sans utiliser de réseaux de neurones, tels que des réseaux de neurones convolutionnels, ou des réseaux de neurones multicouches.

**6.** Dispositif (1) selon l'une des revendications précédentes, dans lequel le dispositif (1) est embarqué, et le classifieur (3a) est choisi notamment dans la liste suivante :

- un boosting algorithme ;
- une cascade adaboost ;
- est réalisé par apprentissage actif ;
- un classifieur binaire.

**7.** Dispositif (1) selon l'une des revendications précédentes, dans lequel les caractéristiques de forme de chaque zone segmentée (5a) de pixels binaires sont choisies notamment parmi la liste suivante :

- surface,
- périmètre,
- centroïde de degré 1,
- centroïde de degré 2,
- orientation,
- Calcul des moments d'ordre 1 et 2 ;
- Carré englobant ; et/ou
- Ellipse englobante.

**9.** Dispositif (1) selon l'une des revendications précédentes, dans lequel la segmentation des pixels est réalisée à l'aide d'un seuil binaire fixe.

**10.** Dispositif (1) selon l'une des revendications précédentes 1 à 7, dans lequel la segmentation des pixels est réalisée à l'aide d'un seuil binaire adaptatif en fonction d'une luminance.

**11.** Dispositif (1) selon l'une des revendications précédentes, dans lequel le calcul de l'image monochromatique est réalisé en nuance de gris, ladite nuance de gris étant fonction de la valeur des canaux de couleur (R, V, B) des pixels couleurs.

**12.** Dispositif (1) selon l'une des revendications précédentes, dans lequel la classe est choisie parmi la liste suivante :

- marche ;
- course ;
- levée ;
- assise.

**13.** Dispositif (1) selon l'une des revendications précédentes, dans lequel la classe est choisie parmi la liste suivante :

- marche avec un objet porté avec 2 mains ;
- chute ;
- assise sur une chaise ;
- lacer ses lacets ;
- marche Parkinsonienne ;
- assise au sol ;
- ramasser un objet ;
- chercher un objet sous une chaise ;

- lever d'une chaise et marche.

**14.** Dispositif (1) selon l'une des revendications précédentes, dans lequel les moyens de stockage (4) sont configurés pour stocker les séquences successives de classe obtenues, et déterminer une carte d'activité du sujet.

**15.** Dispositif (1) selon l'une des revendications précédentes, dans lequel le radar (2) émet les signaux radar selon une bande de fréquence comprise entre 6 MHz et 250 GHz, de préférence entre 2 et 4 GHz, de façon plus préférée entre 2,3 et 2,5 GHz.

**16.** Système de contrôle de l'actimétrie d'un sujet, ledit système comprenant :

- un bâtiment avec des murs ;
- un dispositif (1) selon l'une quelconque des revendications 1 à 15, ledit dispositif étant intégré dans un mur dudit bâtiment,

**17.** Système selon la revendication précédente, dans lequel le dispositif (1) est couplé avec un dispositif d'alerte et de contrôle, apte à alerter d'une nécessité d'intervention sur le sujet dont l'actimétrie est suivie, si la succession de classes des images binaires (5), déterminée en temps réel, indique comme carte d'activité un comportement à risque.

**18.** Système selon l'une des revendications 16 ou 17, dans lequel le dispositif comporte :

- un ou plusieurs radars monostatiques ; et/ ou
- un ou plusieurs radars bistatiques.

Figure 1

EP 3 805 785 A1

1

4

Action: chute, etc..

Ondes radio émises

Chemin signal fréquence radio transmis

Chemin signal fréquence transmis

Données pré-processées

3a

3

Classe 1

Classe 2

Antenne de transmission

Extrémité radar

Cœur numérique

Signal pré-processeur

Algorithme Classification

2

Antenne de réception

Ondes radio réfléchies

Chemin signal radio reçu

Chemin signal fréquence reçu

Chemin signal reçu numérisé

Classe n-1

Classe n

Figure 2

# Traitements

Image micro-doppler — 6    Figure 3

Image binaire

Extraction des caractéristiques :
Surface,
périmètre,
orientation,
Centroïde ordre 1, ordre 2
etc...

Classifieur SVM entrainé

Prédiction classe

Figure 4

**Démonstrateur de laboratoire**

BDD →

Classe 1: chute
Classe 2: cherche un objet sous une chaise
Classe 3: parkinson
Classe 4: prendre un objet et le soulever
Classe 5:assise au sol
Classe 6: assise sur une chaise
Classe 7: lacer ses lacets
Classe 8:TUG
Classe 9: marche
Classe 10: marche avec objet porté

## Figure 5

Europäisches
Patentamt

European
Patent Office

Office européen
des brevets

**RAPPORT DE RECHERCHE EUROPEENNE**

Numéro de la demande

EP 19 30 6308

## DOCUMENTS CONSIDERES COMME PERTINENTS

| Catégorie | Citation du document avec indication, en cas de besoin, des parties pertinentes | Revendication concernée | CLASSEMENT DE LA DEMANDE (IPC) |
|---|---|---|---|
| X | HE MI ET AL: "Human Fall Detection Based on Machine Learning Using a THz Radar System", 2019 IEEE RADAR CONFERENCE (RADARCONF), IEEE, 22 avril 2019 (2019-04-22), pages 1-5, XP033616856, DOI: 10.1109/RADAR.2019.8835828 [extrait le 2019-09-12] | 1-14, 16-18 | INV. G01S7/41 A61B5/00 G01S13/88 |
| Y | * pages 3-4 * | 12,13,15 | |
| | ----- | | |
| X | WU QISONG ET AL: "Radar-based fall detection based on Doppler time-frequency signatures for assisted li", IET RADAR SONAR NAVIGATION, THE INSTITUTION OF ENGINEERING AND TECHNOLOGY, UK, vol. 9, no. 2, 1 février 2015 (2015-02-01) , pages 164-172, XP006051698, ISSN: 1751-8784, DOI: 10.1049/IET-RSN.2014.0250 * pages 166-168 * | 1-14, 16-18 | |
| | ----- | | DOMAINES TECHNIQUES RECHERCHES (IPC) |
| Y | JAVIER RIOS JESUS ET AL: "Application of Linear Predictive Coding for Human Activity Classification Based on Micro-Doppler Signatures", IEEE GEOSCIENCE AND REMOTE SENSING LETTERS, IEEE SERVICE CENTER, NEW YORK, NY, US, vol. 11, no. 10, 1 octobre 2014 (2014-10-01), pages 1831-1834, XP011548152, ISSN: 1545-598X, DOI: 10.1109/LGRS.2014.2311819 [extrait le 2014-05-12] | 12,13,15 | G01S A61B |
| A | * le document en entier * | 1 | |
| | ----- | | |
| | -/-- | | |

Le présent rapport a été établi pour toutes les revendications

| Lieu de la recherche | Date d'achèvement de la recherche | Examinateur |
|---|---|---|
| Munich | 1 avril 2020 | Kern, Olivier |

CATEGORIE DES DOCUMENTS CITES

X : particulièrement pertinent à lui seul
Y : particulièrement pertinent en combinaison avec un
   autre document de la même catégorie
A : arrière-plan technologique
O : divulgation non-écrite
P : document intercalaire

T : théorie ou principe à la base de l'invention
E : document de brevet antérieur, mais publié à la
   date de dépôt ou après cette date
D : cité dans la demande
L : cité pour d'autres raisons

& : membre de la même famille, document correspondant

EPO FORM 1503 03.82 (P04C02)

page 1 de 2

Europäisches
Patentamt

European
Patent Office

Office européen
des brevets

**RAPPORT DE RECHERCHE EUROPEENNE**

Numéro de la demande

EP 19 30 6308

| | **DOCUMENTS CONSIDERES COMME PERTINENTS** | | | |
|---|---|---|---|
| Catégorie | Citation du document avec indication, en cas de besoin, des parties pertinentes | Revendication concernée | CLASSEMENT DE LA DEMANDE (IPC) |
| A | FIORANELLI FRANCESCO ET AL: "Bistatic human micro-Doppler signatures for classification of indoor activities", 2017 IEEE RADAR CONFERENCE (RADARCONF), IEEE, 8 mai 2017 (2017-05-08), pages 610-615, XP033104089, DOI: 10.1109/RADAR.2017.7944276 [extrait le 2017-06-07] * le document en entier * ----- | 1 | |
| | | | DOMAINES TECHNIQUES RECHERCHES (IPC) |

Le présent rapport a été établi pour toutes les revendications

| Lieu de la recherche | Date d'achèvement de la recherche | Examinateur |
|---|---|---|
| Munich | 1 avril 2020 | Kern, Olivier |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons
 
& : membre de la même famille, document correspondant

EPO FORM 1503 03.82 (P04C02)

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Littérature non-brevet citée dans la description**

- **M. G. AMIN ; Y. D. ZHANG ; F. AHMAD ; K. C. D. HO.** Radar 2 Signal Processing for Elderly Fall Détection: The future for in-home monitoring. *IEEE Signal Processing Magazine,* 2016, vol. 33 (2), 71-80
- *Report to Congress: Aging services Technology Study,* 2012
- **C. DEBES ; A. MERENTITIS ; S. SUKHANOV ; M. NIESSEN ; N. FRANGIADAKIS ; A. BAUER.** Monitoring Activities of Daily Living in Smart Homes: Understanding human behavior. *IEEE Signal Processing Magazine,* 2016, vol. 33 (2), 81-94
- **H. LI, A. ; SHRESTHA, F. ; FIORANELLI, J. ; L. KERNEC ; H. HEIDARI.** Multisensory Data Fusion for Human Activities Classification and Fall Détection. *IEEE Sensors,* 2017
- **E. CIPPITELLI ; F. FIORANELLI ; E. GAMBI ; S. SPINSANTE.** Radar 2 and RGB-Depth Sensors for Fall Détection: A Review. *IEEE Sensors Journal,* 2017, vol. 17 (12), 3585-3604
- **B. JOKANOVI ; M. AMIN.** Fall Détection Using Deep Learning in Range-Doppler Radar 2s. *IEEE Transactions on Aerospace and Electronic Systems,* 2017, vol. PP (99), 1-1
- **NOBUYUKI OTSU.** A threshold selection method from gray-level histograms. *IEEE Trans. Sys., Man., Cyber.,* 1979, vol. 9, 62-66